# EUROPEAN PATENT SPECIFICATION

(11) **EP 0 477 359 B1**
(45) Date of publication and mention of the grant of the patent: **05.07.1995**
(21) Application number: 91909241.1
(22) Date of filing: 15.04.1991
(51) Int. Cl.: C07C 35/14, C07C 29/17, C07C 29/10, C07C 29/48, C07C 43/196, C07C 41/26, C07C 41/03

(54) **SYNTHESIS OF CYCLITOLS FROM SUBSTITUTED ARENE DIOLS**
SYNTHESE ION CYCLITOLEN AUS SUBSTITUIERTEN ARENDIOLEN
SYNTHESE DE CYCLITOLS A PARTIR DE DIOLS D'ARENE SUBSTITUES

(30) Priority: 16.04.1990 US 509341; 31.12.1990 US 636396
(43) Date of publication of application: 01.04.1992
(73) Proprietor: VIRGINIA TECH INTELLECTUAL PROPERTIES, INC., Blacksburg, VA 24060 (US)
(72) Inventor: Hudlicky, Thomas, Blacksburg, Virginia 24060 (US)
(74) Representative: Nicholls, Kathryn Margaret
(86) International application number: PCT/US91/02594
(87) International publication number: WO 91/16290

(56) References cited:
- JOURNAL OF THE AMERICAN CHEMICAL SOCIETY, vol. 112, no. 25, 5 December 1990, pages 9439-9440, American Chemical Society, T. HUDLICKY et al.."Efficient and enantiodivergent synthesis of (+)- and (-)-pinitol", see the whole article
- THE JOURNAL OF ORGANIC CHEMISTRY, vol. 55, no. 15, 20 July 1990 pages 4683-4687, American Chemical Society, T. HUDLICKY et al.
- TETRAHEDRON, vol. 45, no.11, 1989, pages 3463-3476, Pergamon Press, S.V. LEY et al.
- TETRAHEDRON LETTERS, vol. 30, no. 23, 1989, pages 3113-3116, H.A.J.CARLESS et al.
- TETRAHEDRON LETTERS, vol. 31, no. 9, 1990, pages 1323-1326, H.SECEN et al.

## Description

### BACKGROUND OF THE INVENTION

The present invention relates to the synthesis of cyclitols from substituted arene diols.

Cyclitols are polyoxyfunctional (i.e., having 2 to 6 hydroxy, alkoxy, aryloxy or phosphate functionalities) cyclohexane derivatives, and as such are classified as carbohydrates [Anderson, In The Carbohydrates Chemistry and Biochemistry, Pigman et al., Eds., Academic Press: New York, Vol. Ia, Chap. 15, 1972]. All possible stereoisomers of these compounds are known to occur in nature (seven meso forms and one DL pair). Indeed, cyclitols appear to be present, both free and combined, in the tissues of nearly all living species [Posternak, T. The Cyclitols, Hermann: Paris, 1962]. Dihydroconduritols are a subclass of inositols in which the number of hydroxyl groups present is reduced to four. These compounds, along with conduritols (cyclohexenetetrols) are of interest because of their potential use as inhibitors for glycosidases [Posternak, T. The Cyclitols, Hermann: Paris, 1962].

Previous syntheses of these types of compounds have started from achiral sources and have either involved numerous steps, incomplete stereospecifity at some point, or did not lead to optically pure products [Posternak et al., Helv. Chim. Acta 36:251, 1953; Gorin, Can. J. Chem. 42:1749, 1964; Nakajima et al., S. Ber. 92:173, 1959]. Benzene-cis-diol has been used in a number of natural product syntheses [Carless et al. Tetrahedron Lett. 30:3113, 1989; Carless et al., Tetrahedron Lett. 30:1719, 1989]. Ley et al. used the benzene diol to synthesize (+)-pinitol [Tetrahedron Letters 45:3463, 1989] and myo-inositol triphosphate [Tetrahedron Letters 29:5305, 1988]. One obvious disadvantage to using benzene-cis-diol in natural product synthesis, however, is the preclusion of enantiocontrolled synthesis without further (usually enzymatic) manipulations of the meso intermediates. For this and other reasons, the methods of the present invention utilize optically pure chiral synthons resulting from microbial oxidation of monosubstituted benzene derivatives.

Other syntheses of cyclitols are disclosed in Secen et al., Tetrahedron Lett. 31:1323, 1990; Akbulut et al., J. Org. Chem. 53:3338, 1988; Sutbeyaz et al., J. Chem. Soc. Commun. 1330, 1988; Bruce et al., Tetrahedron Lett. 30:7257, 1989; Kobayashi et al., J. Org. Chem. 55:1169, 1990; and Gorin, Canadian J. chem. 42:1748, 1964.

In 1970 Gibson and coworkers reported the enantioselective oxidation of toluene to cis-toluenediol [(+)-cis-2,3-dihydroxylmethylcyclohexa-4,6-diene] by a mutant of Pseudomonas putida, a soil bacterium [Gibson et al., J. Biochemistry 9:1626 (1970)]. Since that time many other simple arenes were shown to yield diols of this type through microbial oxidation techniques [Jerina et al., Arch. Biochem. Biophys. 142:394 (1971); Gibson et al., Biochemistry 7:3795 (1968); Jeffrey et al., Biochemistry 14:575 (1975); Burlingame et al., J. Bacteriol. 168:55 (1986); Gibson et al., Biochem. Biophys. Res. Commun. 50:211 (1973); Gibson et al., J. Bacteriol. 119:930 (1974); Whited et al., J. Bacteriol. 166:1028 (1986); Ziffer et al., Tetrahedron 33:2491 (1977)].

Despite the operational simplicity and complete stereospecificity of the reaction producing such diols, little use of this transformation has been made in organic synthesis, save for a few recent reports. Hudlicky et al. [J. Am. Chem. Soc. 110:4735 (1988)] used the toluene diol to synthesize enones useful for prostaglandin synthesis, aldehydes which are potential synthons for perhydroazulene terpenes, and cyclohexene oxide which is the descarbobenzoxy derivative of crotepoxide. Hudlicky et al. [J. Org. Chem. 54:4239 (1989)] have also used the styrene diol to synthesize zeylena, another cyclohexene oxide. However, the full potential of such diols for synthesis of chiral synthons has not yet been fully realized.

### SUMMARY OF THE INVENTION

The present invention takes advantage of the chirality introduced in the microbial oxidation of arene diols to simply and efficiently produce cyclitols. A short enantioselective approach to cyclitols is disclosed. One key step in the synthetic approach is the use of chiral arene diols produced by microbial oxidation of arenes. In such diols, while the molecule is suitably modified to allow for face selectivity in subsequent hydroxylation sequences. Thus, a chiral cyclitol has been obtained from an achiral aromatic source.

The methods of the present invention are relatively simple and more economical than prior methods for such syntheses. A further advantage is provided by such methods in that cyclitols can be produced from C1 substituted arenes (such as halobenzenes, particularly chlorobenzene, toluene, styrene) which are usually considered to be useless, sometimes toxic, waste products. Thus, waste can be converted into useful sugars in accordance with the present invention. These and other advantages of the present invention will be apparent to those skilled in the art from the disclosure herein.

### DESCRIPTION OF THE FIGURES

Figure 1 is a generalized scheme for control of stereochemistry in synthesis of cyclitols from chiral arene diols.

Figure 2 is a scheme for functionalization of a phosphate cyclitol derivative.

Figure 3 is a scheme for the synthesis of dihydroconduritol C (1) and conduritol C (45).

Figure 4 is a scheme for the synthesis of Conduritol F (14) from chlorobenzene-cis-diol (5).

Figure 5 is a scheme for the synthesis of (-)-pinitol (17).

Figure 6 is a scheme for the synthesis of (+)-pinitol (21).

### DETAILED DESCRIPTION OF THE INVENTION

Cyclitols can be synthesized in accordance with the present invention from appropriately substituted chiral arene diols. Arene diols of the general formula (I): 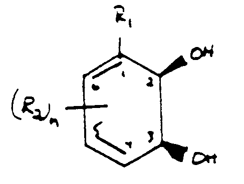
can be used in the methods of the present invention. R1 can be a halogen (particularly chloro and bromo), lower alkyl (particularly methyl) or lower alkenyl (particularly methenyl). R2 is halogen where n is from 0 to 3. Preferably, R1 is chloro and n is 0 such that the arene is chlorobenzene. R2 can also be a group or structure bridging two arene moieties, such as without limitation the bridging carbon bond in polychlorobiphenyls, the bridging methylene groups in polystyrene, and bridging oxygen in polyaryl ethers. Appropriate values for the various R groups will depend upon the desired cyclitol product. Since the arene diols are synthesized by microbial oxidation, substitution of the arene diol is limited only by the microbial process (i.e., by the substituted arenes which can serve as a substrate for the microbial process).

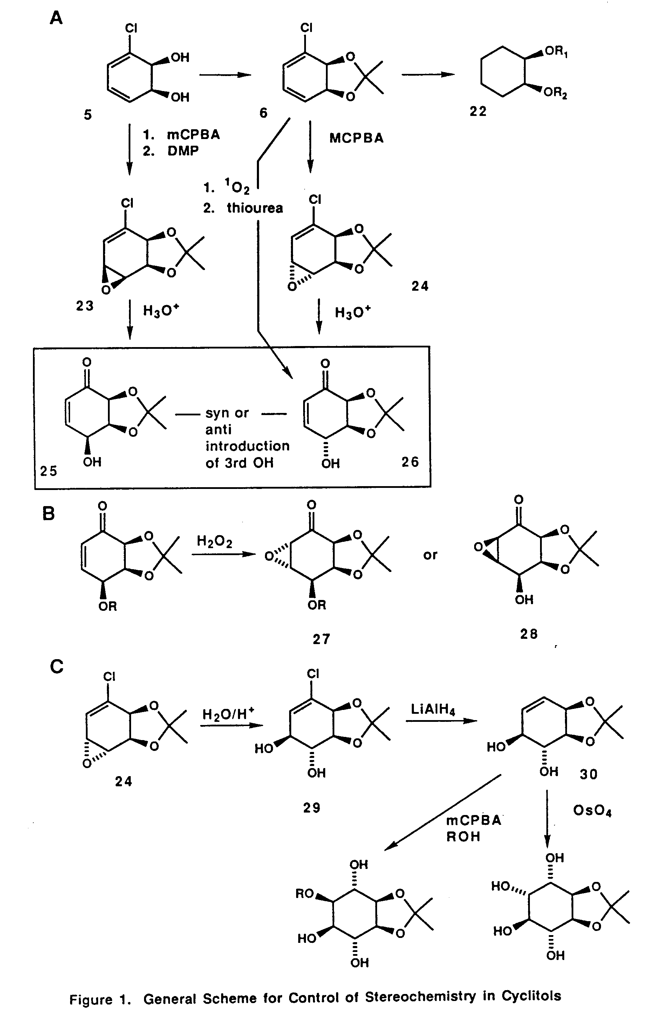

A generalized scheme for synthesis of cyclitols from arene diols is shown in Figure 1. Although the chlorinated diol is shown in Figure 1, the same general scheme can be used with other substituted arene diols of formula (I) above.

Cyclitols having two functional groups (22) can be produced by full reduction of the acetonide (6) using appropriate reagents to give the desired R groups.

Cyclitols having three functional groups (25 and 26) can be produced by acidic addition of water or an alcohol (ROH) to epoxides (23 and 24, respectively). Epoxides (23 and 24) are formed from the diol (5) and the acetonide (6), respectively, depending on whether syn or anti addition of the third functional group is desired. Use of an alcohol (ROH) rather than water yields-OR at C4. Anti addition of the third functional group can also be achieved directly from the acetonide (6) by sequential treatment with singlet oxygen and thiourea.

Additional functional groups can be added by converting an enone to an epoxide by treatment with peroxide as shown in Figure 1B. Addition of the epoxide functionality is anti if the group on the preceding carbon is protected (i.e., -OR) or syn if the C4 group is unprotected (i.e., -OH). For example, if C4 bears -OCH₃, additional of the epoxide function is anti. Treatment of the epoxide with acidic water or alcohol (ROH) as shown in Figure 1C provides -OH or -OR functionality at the epoxide carbons (29). After further reduction, the resulting protected tetrol (30) can be further functionalized by treatment with either osmium tetroxide (syn addition, 31) or MCPBA and acidic alcohol (ROH) (anti addition, 32).

Within the context of this general scheme relative stereochemistry can be controlled at a given step by controlling the character of the functional group on the preceding carbon. For example, if a given step will add functionality to C4 and C5, relative stereochemistry can be controlled by the character of the functionality on C3. If the preceding functionality is -OH, the next functionality will be added syn; if the preceding functionality is protected (i.e., -OR), the next functionality will be added anti. Similarly, use of acidic ROH instead of acidic water to add functionality avoids making symmetrical intermediates which lead to meso compounds and loss of control of stereospecificity in the reaction.

Since only the syn or anti distinction is used throughout the general scheme, each center can be formed either "up" or "down" at will by using the variables disclosed herein. If the original diol needs to be trans in order to achieve the desired configuration at C2 and C3, either carbon center can be inverted using the Mitsunobu process described in Hudlicky et al., J. Org. Chem. 54:4239, 1989.

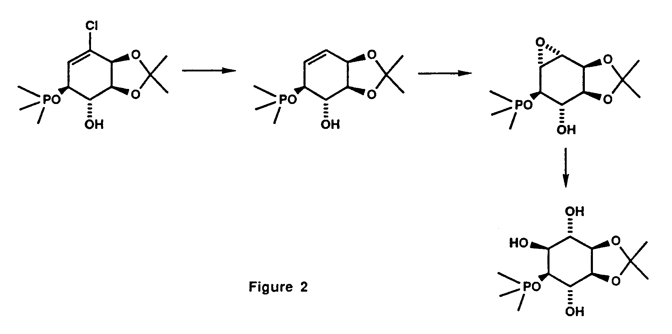

Phosphate derivatives of cyclitols can also be produced by functionalizing the appropriate phosphoric acid derivatives, for example as shown in Figure 2.

It will be apparent to those skilled in the art that radioactively labelled cyclitols can also be prepared in accordance with the present invention by using radio-labelled reagents at appropriate stages in the synthetic process.

All hydrolytic reactions were carried out in a nitrogen or argon atmosphere, with standard techniques for the exclusion of air and moisture. Glassware used for moisture-sensitive reactions was flame-dried with an internal inert gas sweep. THF, ethyl ether, DME and benzene were distilled from benzophenone ketyl; dichloromethane and toluene from calcium hydride.

### Example 1

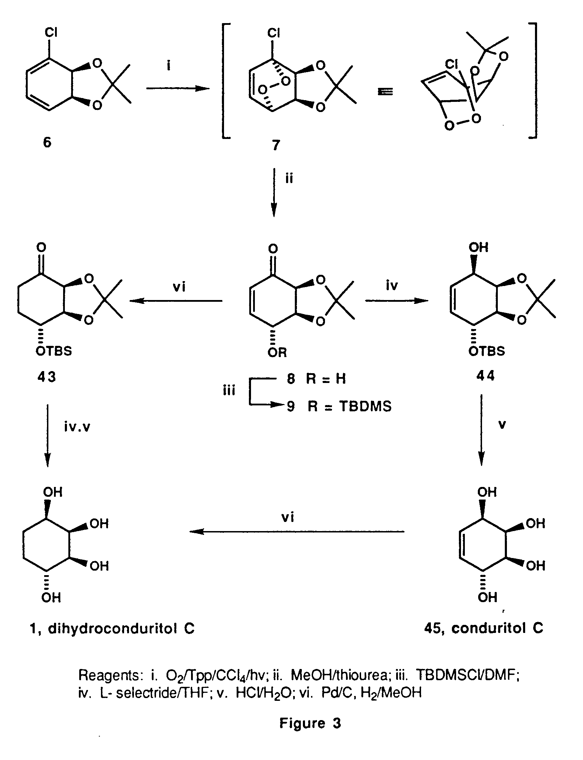

The synthesis of dihyroconduritol C (1) from chlorobenzene-cis-diol (5) is shown in Figure 3. Protection of chlorobenzene-cis-diol (5) as an acetonide (6) was followed by reaction of the diene with singlet oxygen to afford endoperoxide (7). Only one regioisomer of endoperoxide (7) was formed, with attack occurring from the less hindered alpha-face. The stereospecific reaction of singlet oxygen with chiral dienes such as acetonide (6) was first reported by Hudlicky et al. [J. Am. Chem. soc. 108:5655, 1988]. Reduction of endoperoxide (7) with thiourea resulted in conversion to the hydroxyenone (8). Alternatively, it is unnecessary to isolate the endoperoxide (7) prior to reduction. Instead in a one-pot sequence the required alpha-hydroxyl at C4 of Conduritol C was introduced along with the ketone functionality at C1. Hydrogenation of the double bond in hydroxyenone (8) proceeded smoother and in higher yields if the alcohol was first protected as the tert-butyldimethylsilyl ether (9) or tert-butylsilyl ether (43). Reduction of the protected ketone with L-selectride proceeded with complete stereoselectivity to afford an intermediate alcohol possessing the correct relative and absolute stereochemistry of the four chiral centers of Conduritol C. Deprotection of intermediate alcohol under acidic conditions gave Conduritol C (1), whose spectral data, [a]_{D}, mp, and ¹³C NMR were in agreement with literature values [Gorin et al. 27:325, 1973].

In one experimental sequence Conduritol C was synthesized from the acetonide (6) as follows:

(1R,4S,5R,6S)-1-Chloro-7,8-dioxa-5,6-di-O-isopropylidenebicyclo [2.2.2]oct-2-ene (7)

Tetraphenylporphine (3 mg) was dissolved in 5 mL of chloroform, 10 min before initiation of the reaction; this solution was transferred to the reaction vessel ( a water jacketed flask, with 2 necks and a glass frit bubbler attached) using 50 mL of CCl₄. The acetonide 6a (736 mg, 4.65 mmol) was added to the reaction vessel using 50 mL of CCl₄. The temperature of the reaction mixture was kept ^{~}20 °C. Oxygen was bubbled through the solution while irradiated with a quartz lamp (500 W). The reaction was monitored by TLC (silica gel, hexane-ethyl acetate, 9:1) and was complete in 2.5 h. The solvent was removed under reduced pressure without heating. The dark green residue was taken up in 80 mL of hexane and shaken for 5 min with 0.5 g of charcoal; the mixture was filtered through celite. Evaporation of the solvent afforded 950.9 mg (4.32 mmol, 93%) of clean endoperoxide as light brown crystals, which could be used without further purification (mp=69-71°C). An analytical sample was obtained by sublimation (<30 °C, 0.005 torr) mp=74-76 °C.

(4R,5S,6R)-4-Hydroxy-5-6-di-O-isopropylidenecyclohex-2-en-1-one (8)

To 951 mg (4.36 mmol) of endoperoxide 7 in 6 mL of MeOH at 10 °C was added 364 Mg (4.79 mmol, 1.1 eq) of thiourea in 5 mL of MeOH dropwise. After the addition was complete, the reaction mixture was stirred at room temperature for 0.5 h; the solvent was evaporated under reduced pressure at room temperature. The residue was quickly diluted with 50 mL of ethyl ether, and the precipitate produced was filtered through celite. The filtrate was evaporated to produce a viscous yellow oil. The crude product was purified by flash chromatography (silica gel, methylene chloride-acetone, 8:2) to afford 680 mg (3.71 mmol, 85%) of a light yellow oil, which slowly solidified: mp=72-72.5 °C.

(4R,5S,6R)-4-O-(t-butyldimethylsilyl)-5,6-di-O- isopropylidenecyclohex-2-en-1-one (9)

To a solution of 1.00 g (6.67 mmol, 3 eq), of t-butyldimethylchlorosilane in 4 mL of dry DMF, 1.5 mL of diisopropylethylamine (8.58 mmol, 3.8 eq.) was added at room temperature. After the hydrochloric acid was removed by the stream of Argon, the enone 8 (415.4 mg, 2.258 mmol) in 4 mL of dry DMF was added dropwise. The reaction mixture was stirred at room temperature for 8.5 h.; then 10 mL of brine and 20 mL of ethyl acetate were added, the stirring was continued for 10 min. The layers were separated and the aqueous layer was washed with ethyl acetate (2 x 4 ml). The combined organic fractions were washed with brine (3 x 5 mL), dried over sodium sulphate, and solvent was removed under reduced pressure affording a dark oil which was immediately purified by flash chromatography (silica gel, ethyl acetate-hexane, 3:7) producing 579.5 mg (5.74 mmol, 86%) of a viscous oil which solidified upon refrigeration (mp=50-54 °C). This product was suitable for the next reaction. An analytical sample was obtained by distillation of the solid (Kugelrohr, 100-110 °C/0.1 torr). mp=56.6-57 °C.

(2S,3S,4R)-4-O-(t-butyldimethylsilyl)-2,3-di-O- isopropylidenecyclohexan-1-one (43)

To a solution of 116 mg (0.374 mmol) of enone 9 in MeOH (6 mL) was added 9.5 mg of 10% Pd/C. The mixture was hydrogenated (50 psi) in a Parr vessel at room temperature for 5.5 h. The reaction mixture was filtered through celite; evaporation of the solvent produced 109 mg (97%) of a colorless liquid, which crystallized upon refrigeration. This product was used without further purification. An analytical sample was obtained by distillation (Kugelrohr; 100°/0.2 torr). mp. 44.5-45 °C.

### (-)-Dihydroconduritol C (1)

To 255 mg (0.89 mmol) of ketone 43 in THF (4 mL) was added 1.8 Ml (1.8 mmol) of a 1.0 M solution of L-selectride in THF at 0 °C. The reaction was allowed to warm to room temperature and stirred for 3 h. The reaction was quenched by the addition of H₂O (1 mL), EtOH (3 mL), 3 N aq NaOH (4 mL), and 30% aq H₂O₂ (3 mL). The aqueous phase was saturated with K₂CO₃ and extracted with EtOAc (5 x 20 mL). The organic phase was dried (MgSO₄), filtered, and concentrated leaving a yellow oil which after purification by flash chromatography (silica gel, hexane-EtOAc, 1:1) afforded 193 mg (68 mmol, 76%) of pure alcohol.

### (1R,2R,3R,4R)-1-hydroxy-2,3-di-O-isopropylidene-4-O-(t-butyldimethylsilyl)cyclohexane.

To 35 mg (0.12 mmol) of the resulting alcohol in 2 mL of a 1:1 mixture of acetone-H₂O, was added dropwise concentrated HCl until pH=2. The solution was stirred at room temperature for 8 h. Solvent and HCl were removed under vacuum leaving 16 mg (0.11 mmol, 92%) of pure tetrol whose spectral data were in agreement with literature values.

### Example 2

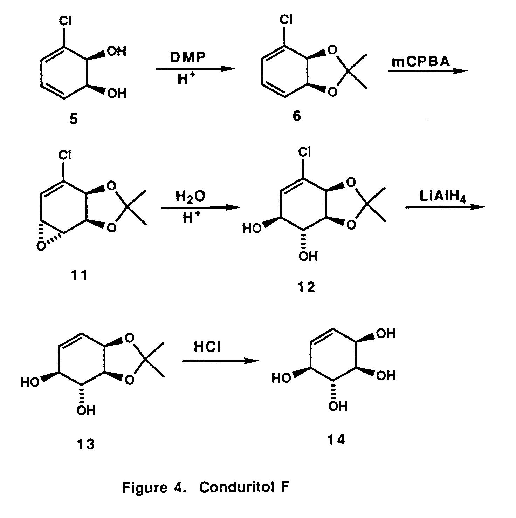

Synthesis of Conduritol F (14) is shown in Figure 4. Acetonide (6) is converted to epoxide (11) by treatment with m-chloroperbenzoic acid ("MCPBA"). Acid treatment of the epoxide (11) yields protected chlorinated tetrol (12) which is reduced to protected tetrol (13). Deprotection of the protected tetrol (13) under acid conditions produces Conditurol F (14).

(1R,4S,5S,6R)-3-Chloro-2,3-di-O-isopropylidene-7-oxa- bicyclo[4.1.0]hept-2-ene (11a)

To 1.915 g (10.3 mmol) of diene 6a in CH₂Cl₂ (75 mL) at 0 °C was added 1.78 g (8.2 mmol) of mCPBA portionwise. The solution was allowed to warm to room temperature and stirred for 8 h. The solution was washed with 15% aq sodium sulfite (2 x 50 mL), saturated aq sodium bicarbonate (2 x 50 mL), and water (50 mL), then dried (MgSO₄), filtered, and concentrated. Unreacted starting material was removed under vacuum leaving 1.50 g (7.3 mmol 89%) of pure epoxide 11a as a colorless solid: mp = 59-60 °C.

(1R,4S,5S,6R)-3-Bromo-2,3-di-O-isopropylidene-7-oxa- bicyclo[4.1.0]hept-2-ene (11b)

To a solution of acetonide 6b (1.0g, 4.33 mmol) in CH₂Cl₂ (40 mL) was added mCPBA (85%, 1.1g, 1.2 eq) in one portion. The mixture was stirred at room temperature for 12 h. The mixture was filtered, the filtrate was treated with 10% aqueous sodium sulfite (5 mL) and separated. The organic layer was washed with H₂O (10 mL, 10% aq NaOH (10 mL) and brine (10 mL) and dried over Na₂SO₄. The solvent was evaporated under reduced pressure. The crude product was purified by flash chromatography (silica gel, hexane-ethyl acetate, 4:1) to afford colorless crystals (871 mg, 82%): mp 78-78.5 °C.

### Example 3

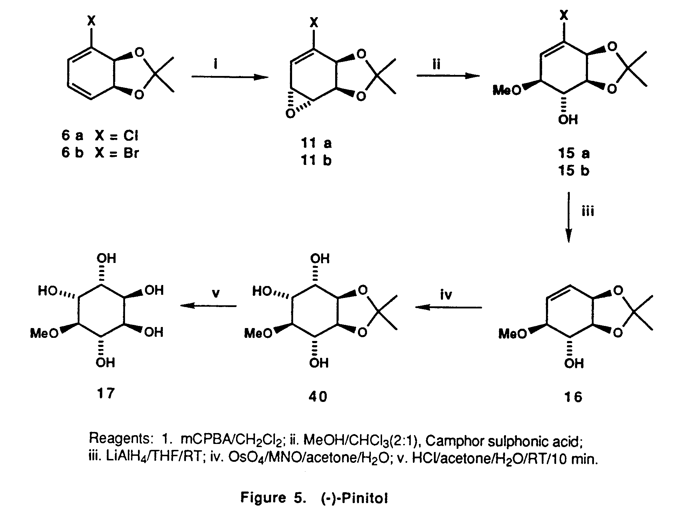

Synthesis of (-)-pinitol (17) is shown in Figure 5. The C5 methoxy functionality is added by treatment of epoxide (11) with methanol under acidic conditions to produce intermediate methylether (15), which is then reduced producing protected methoxy triol (16). (-)-pinitol (17) is produced by treatment of the protected methoxy triol (16) with osmium tetroxide to introduce hydroxyl functionality at C1 and C6 (as in 40) and with acid to deprotect the hydroxyl groups at C2 and C3.

(3R,4R,5S,6S)-1-Chloro-4-hydroxy-5,6-di-O-isopropylidene-3-O- methylcyclohex-1-ene (15a)

To 1.03 g (5.0 mmol) of epoxide 11a in MeOH (16 mL) and CHCl₃ (8 mL) was added 183 mg of CSA. The mixture was stirred at room temperature for 45 min. The solution was poured into CH₂Cl₂ (50 mL), washed with saturated aq NaHCO₃ (2 x 50 mL), and H₂O (50 mL), then dried (MgSO₄), filtered, and concentrated leaving a yellow oil which after purification by flash chromatography (silica gel, hexane-EtOAc, 1:1) afforded 800 mg (4.3 mmol, 86%) of methyl ether 15a as an oil.

(3R,4R,5S,6S)-1-Bromo-4-hydroxy-5,6-di-O-isopropylidene-3-O- methylcyclohex-1-ene (15b)

To a solution of 750 mg (3.036 mmole) of epoxide 11b in 25 mL of MeOH-CHCl₃ (2:1), was added 133 mg (0.572 mmole, 0.2 eq.) of camphorsulfonic acid. The reaction mixture was stirred at room temperature for 45 min.; then the reaction was concentrated and extracted with ethyl acetate (2 x 20 mL). The organic layer was washed with saturated sodium bicarbonate solution (10 mL), dried over Na₂SO₄ and the solvent evaporated under reduced pressure. After purification by flash chromatography (silica gel; hexanes-ethyl acetate, 1:1) 750 mg (89%) of a colorless liquid was obtained.

(3R,4R,5S,6S)-3-O-Methyl-4-hydroxy-5,6-di-O-isopropylidene cyclohex-1-ene (16)

Method A: To a solution of 60 mg (0.216 mmol) of bromoolefin 15b in 3 mL of anhydrous THF, was added LiAlH₄ (8.4 mg, 0.22 mmol) under an Argon atmosphere. The reaction mixture was stirred at room temperature for 8 h. The reaction was quenched with brine and extracted with ethyl acetate (2 x 20 mL). The organic layers were dried over Na₂SO₄, and concentrated leaving a yellow oil. Purification by flash chromatography (silica gel; hexane-ethyl acetate, 1:2) afforded 36.3 mg (0.18 mmol), 85%) of cyclohexene 16 as an oil.
Method B: The above procedure was repeated using vinylchloride 15a and heating to reflux to afford cyclohexene 16 in 54% yield, identical in all respects to the material obtained above.

(1S,2S,3S,4R,5S,6R)-2,3,6-trihydroxy-4,5-di-O-isopropylidene-1- O-methylcyclohexane (40)

To a solution of 150 mg (0.75 mmol) of olefin 16 in 6.8 mL of acetone-H₂O (3:1), was added 67 mg of MNO and 0.2 mL of a 2.5 wt % solution of OsO₄ in t-BuOH at room temperature. The reaction mixture was stirred at room temperature for 2 days, when an additional 0.2 ml of OsO₄ solution was added. The reaction was continued for 3 more days (analysis by TLC showed some starting material left). The reaction mixture was quenched with 10 mL of 15% NaHSO₃ and saturated with NaCl, and was extracted with ethyl acetate (2 x 15 mL). The combined organic layers were dried over Na₂SO₄, and concentrated to afford 171 mg of an oil which crystallized upon refrigeration. Purification by column chromatography (silica gel; hexanes-ethyl acetate 1:5) gave 110 mg (62.7%) of triol 40 and 58 mg of recovered starting material. Triol 40: mp = 81.5°C.

### (-)-Pinitol 17

To a solution of triol 40 (10 mg, 0.043 mmol) in 3 ml of acetone-H₂O (2:1) was added 3 drops of concentrated HCl. The reaction was stirred at room temperature for 10 min. Solvent was removed under vacuum leaving 8.0 mg (0.041 mmol, 96%) of a colorless solid: mp = 184.5-185.0 °C.

### Example 4

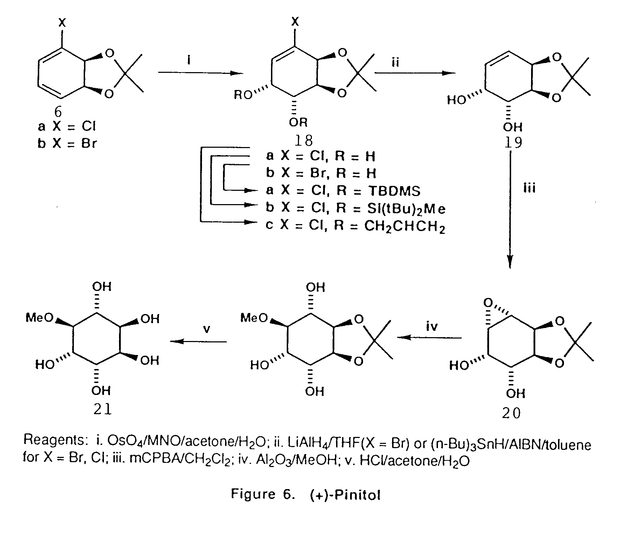

Synthesis of (+)-pinitol (21) is shown in Figure 6. Treatment of acetonide (6) with osmium tetroxide yields protected chlorinated tetrol (18). The chlorine is removed by reduction to produce intermediate protected tetrol (19), which is subsequently converted to epoxide (20) by treatment with MCPBA. (+)-pinitol (21) is produced by treating the epoxide (20) with methanol to introduce the methoxy and hydroxy functionalities at C6 and C1 (as in 41) and with acid to deprotect C2 and C3.

(1R,2R,3S,4S)-5-chloro-1,2-dihydroxy-3,4-di-O- isopropylidenecyclohex-5-ene (18a)

To 1.00 g (5.38 mmol) of diene 6a in a mixture of acetone (50 mL) and water (15 mL) were added 670 mg (5.70 mmol) of MNO and 2 mL of a 2.5% solution of OsO₄ in tert-butanol. The mixture was stirred at room temperature for 24 h. The solution was concentrated under reduced pressure, and acidified with 1 M HCl. Excess OsO₄ was reduced by addition of 190 mL of 15% NaHSO₃ and NaCl as added to give a saturated solution. The resulting solution was extracted with EtOAc (10x50 mL). The organic fraction was dried (MgSO₄), filtered, and concentrated. Purification by flash chromatography afforded 1.06 g (4.84 mmol, 90%) of pure diol 18a as an oil.

(1R,2R,3S,4S)-5-Bromo-1,2-dihydroxy-3,4-di-O-isopropylidene cyclohex-5-ene (18b)

To a solution of acetonide 6b (887 mg, 3.33 mmol) in 58 mL of acetone-H₂O (3:1) were added 630 mg (5.38 mmol) of MNO and 2 mL of a 2.5 wt % of OsO₄ in t-butanol. The solution was stirred at room temperature for 8 h. After concentration, the solution was acidified to pH 8-9 and treated with sodium bisulfite. The aqueous phase was extracted with EtOAc (30 x 10 mL). Purification by flash chromatography (silica gel, EtOAc-hexane, 2:1) afforded 885 mg (3.32 mmol, 97%) of colorless crystals. mp=113-114°C

(1S,2R,3R,4S,5R,6S)-2,3-Dihydroxy-4,5-di-O-isopropylidene-7-oxa- bicyclo[4.1.0]heptane (20)

To a solution of cyclohexene 19 (372 mg, 2.00 mmol) in 20 mL of CH₂Cl₂ was added 610 mg (3.54 mmol) of mCPBA. The mixture was stirred for 24 h at room temperature. The reaction mixture was washed with concentrated aqueous sodium bisulfite and sodium bicarbonate, dried over Na₂SO₄, and concentrated leaving an off white solid. Purification by flash chromatography (10% deactivated silica gel, EtOAc-hexane, 9:1) afforded 355 mg (1.76 mmol, 88%) of colorless crystals.

(1R,2R,3R,4S,5R,6S)-2,3,6-trihydroxy-4,5-di-O-isopropylidene 1- O-methylcyclohexane (41)

To a solution of epoxide 20 (101 mg, 0.50 mmol) in 50 mL of methanol was added 5 g of neutral alumina (dried at 100 °C for 4 h). The mixture was refluxed for 24 h with vigorous stirring. The mixture was filtered and the alumina was washed with hot methanol (2 x 15 mL). The combined organic fractions were evaporated and the residue was purified by flash chromatography (silica gel, EtOAc) affording 63 mg (0.30 mmol, 60%) of colorless crystals; mp = 78.5-79°C.

### (+)-Pinitol (21)

To a solution of triol 41 (13.5 mg, 0.064 mmol) in 3 mL of acetone-H₂O (2:1) were added 3 drops of concentrated HCl. The mixture was stirred at room temperature for 30 min., then concentrated under vacuum to afford (+)-pinitol 21 quantitatively as colorless crystals: mp = 184.5-185_{°}C.

### Example 5

The synthesis of Conduritol is summarized in Figure 3. Conduritol C was synthesized as follows:

(1R,2R,3R,4R)-1-Hydroxy-2,3-di-O-isopropylidene-4-O-(t- butyldimethylsilyl) cyclohex-5-ene (44)

To 107 mg (0.359 mmol) of enone 9 in THF (0.15 mL) at 0 °C was added 0.35 mL of 1.0 M L-selectride (0.350 mmol). The solution was stirred for 50 min. The reaction was quenched by the addition of 1 mL of 10% NaOH followed by 1 mL of 30% H₂O₂; stirring was continued at 0 °C for an additional 20 min. The aq layer was saturated with K₂CO₃ and extracted with EtOAc (4 x 3 mL). the combined organic fractions were dried (Na₂SO₄), filtered, and concentrated leaving a yellow oil, which after purification by flash chromatography (silica gel, hexane-EtOAc, 8:2) afforded 55 mg (0.183 mmol, 51%) of alcohol 44.

### Conduritol C (45)

To a solution of olefin 44 (20 mg, 0.0662 mmol) in 3 mL of acetone-water (2:1) was added concentrated aq HCl (3 drops). The reaction was stirred at room temperature for 18 h and concentrated under vacuum. Purification by flash chromatography (10% deactivated silica gel, CHCl₃-MeOH, 4:1) afforded 8.0 mg (0.054 mmol, 82%) of (-)-conduritol C 45.

All references cited herein are incorporated by reference as if fully set forth.

## Claims

1. A method for producing a diol of formula 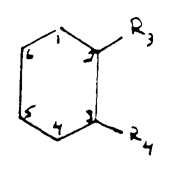 wherein R3 and R4 are hydroxyl or alkoxy, said method comprising the steps of:
(a) providing a substituted arene diol of formula 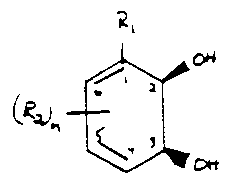 wherein R1 is halogen, lower alkyl or lower alkenyl, R2 is halogen or a bridging group; and n is 0 to 3;
(b) protecting the diol functionality of said substituted arene diol; and
(c) fully reducing said protected substituted arene diol with appropriate reagents to provide R3 and R4.

2. A method for producing a triol of formula 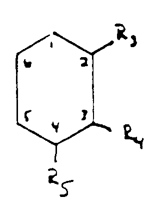 wherein R3, R4, and R5 are hydroxyl or alkoxy, said method comprising the steps of:
(a) providing a substituted arene diol of formula 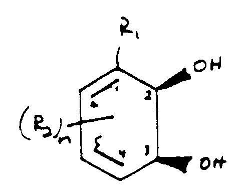 wherein R1 is halogen, lower alkyl or lover alkenyl, R2 is halogen or a bridging group, and n is 0 to 3;
(b) protecting the diol functionality of said substituted arene diol;
(c) forming an epoxide between carbons 4 and 5 of said protected substituted arene diol;
(d) treating said epoxide with acidified water or an appropriate acidified alcohol to provide an enone having R5; and
(e) reducing the functionality at carbon 1 of said enone to hydrogen.

3. A method for producing a triol of formula 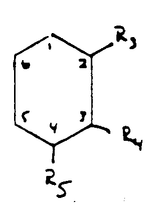 wherein R3, R4, and R5 are hydroxyl or alkoxy, said method comprising the steps of:
(a) providing a substituted arene diol of formula 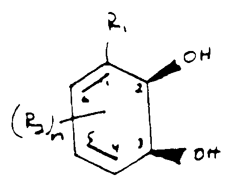 wherein R1 is halogen, lower alkyl or lower alkenyl, R2 is halogen or a bridging group, and n is 0 to 3;
(b) protecting the diol functionality of said substituted arene diol;
(c) forming an endoperioxide between carbons 1 and 4 of said protected substituted arene diol by treatment with singlet oxygen;
(d) treating said endoperioxide with thiourea to form an enone; and
(e) reducing the functionality at carbon 1 of said enone to hydrogen.

4. A method for producing a tetrol of formula 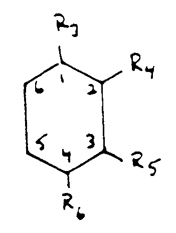 wherein R3, R4, R5, and R6 are hydroxyl or alkoxy, said method comprising the steps of:
(a) providing a substituted arene diol of formula 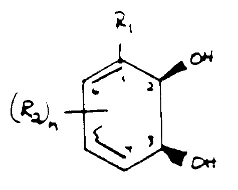 wherein R1 is halogen, lower alkyl or lower alkenyl, R2 is halogen or a bridging group, and n is 0 to 3;
(b) protecting the diol functionality of said substituted arene diol;
(c) forming an endoperioxide between carbons 1 and 4 of said protected substituted arene diol by treatment with singlet oxygen;
(d) treating said endoperioxide with thiourea to form a enone; and
(e) reducing the functionality at carbon 1 of said enone to produce R3.

5. A method of producing a tetrol of formula 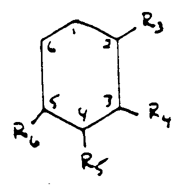 wherein R3, R4, R5 and R6 are hydroxyl or alkoxy, said method comprising the steps of:
(a) providing a substituted arene diol of formula 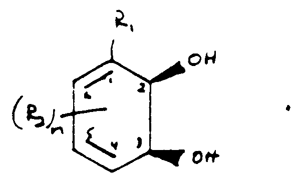 wherein R1 is halogen, lower alkyl or lower alkenyl, R2 is halogen or a bridging group, and n is 0 to 3;
(b) protecting the diol functionality of said substituted arene diol;
(c) converting said protected substituted arene diol to an enone having the keto functionality at carbon 1 and R5 at carbon 4;
(d) forming an epoxide between carbons 4 and 5 of said enone;
(e) treating said epoxide with acidified water or an appropriate acidified alcohol to provide R6; and
(f) reducing the functionality at carbon 1 to hydrogen.

6. A method of producing a tetrol of formula 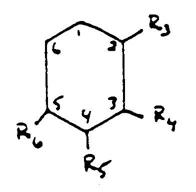 wherein R3, R4, R5, and R6 are hydroxyl or alkoxy, said method comprising the steps of:
(a) providing a substituted arene diol of formula 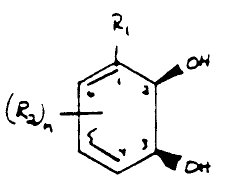 wherein R1 is halogen, lower alkyl or lower alkenyl, R2 is halogen or a bridging group, and n is 0 to 3;
(b) protecting the diol functionality of said substituted arene diol;
(c) forming an epoxide between carbons 4 and 5 of said protected substituted arene diol;
(d) treating said epoxide with acidified water or an appropriate acidified alcohol to provide R5 and R6; and
(e) reducing the functionality at carbon 1 to hydrogen.

7. A method of producing a pentol of formula 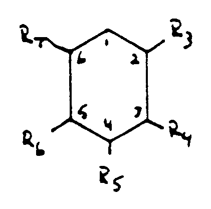 wherein R3, R4, R5, R6, and R7 are hydroxyl or alkoxy, said method comprising the steps of:
(a) providing a substituted arene diol of formula 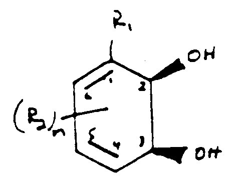 wherein R1 is halogen, lower alkyl or lower alkenyl, R2 is halogen or a bridging group, and n is 0 to 3;
(b) protecting the diol functionality of said substituted arene diol;
(c) forming an epoxide between carbons 5 and 6 of said protected substituted arene diol;
(d) treating said epoxide with acidified water or an appropriate acidified alcohol to provide R6 and R7; and
(e) reducing the functionality at carbon 1 to hydrogen.

8. A method of producing a pentol of formula 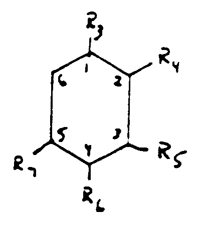 wherein R3, R4, R5, R6 and R7 are hydroxyl or alkoxy, said method comprising the steps of:
(a) providing a substituted arene diol of formula 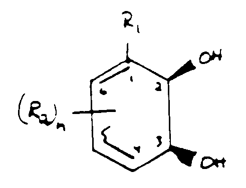 wherein R1 is halogen, lower alkyl or lower alkenyl, R2 is halogen or a bridging group, and n is 0 to 3;
(b) protecting the diol functionality of said substituted arene diol;
(c) forming an epoxide between carbons 5 and 6 of said protected substituted arene diol;
(d) treating said epoxide with acidified water or an appropriate acidified alcohol to provide R7; and
(e) reducing the functionality at carbon 1 to produce R3.

9. A method of producing a hexol of formula 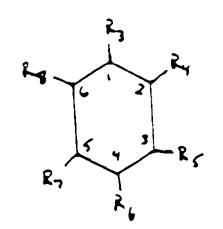 wherein R3, R4, R5, R6, R7, and R8 are hydroxyl or alkoxy, said method comprising the steps of:
(a) providing a substituted arene diol of formula 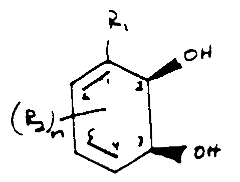 wherein R1 is halogen, lower alkyl or lower alkenyl, R2 is halogen or a bridging group, and n is 0 to 3;
(b) protecting the diol functionality of said substituted arene diol;
(c) forming an epoxide between carbons 4 and 5 of said protected substituted arene diol;
(d) treating said epoxide with acidified water or an appropriate acidified alcohol to provide a protected tetrol having R6 and R7;
(e) reducing the functionality at carbon 1 of said protected tetrol to hydrogen; and
(f) either (1) forming an epoxide between carbons 1 and 6 of said protected tetrol and treating said epoxide with acidified water or an appropriate acidified alcohol to provide R3 and R8, or (2) treating said reduced protected tetrol with osmium tetroxide to provide R3 and R8.

10. The method of claim 1, 2, 3, 4, 5, 6, 7, 8, or 9 wherein said substituted arene diol is of formula

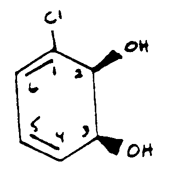

11. The method of claim 1, 2, 3, 4, 5, 6, 7, 8, or 9 wherein one or more intermediate cyclitols employed in the process is converted to a phosphate derivative before proceeding with subsequent steps of the method.

12. The method of claim 1, 2, 3, 4, 5, 6, 7, 8, or 9 wherein one or more of the reagents used in the method are radioactive and wherein the cyclitol produced by said method is radioactive.

13. A method of producing Conduritol C, said method comprising the steps of:
(a) treating (2R, 3S)-2,3-O-isopropylidene-1-chlorocyclohexa-4,6-diene with singlet oxygen to produce (1S, 2S, 3S, 4R)-1-chloro-2,3-O-isopropylidene-5,6-dioxabicyclo[2.2.2]octa-7-ene;
(b) treating said (1S, 2S, 3S, 4R)-1-chloro-2,3-O-isopropylidene-5,6-dioxabicyclo[2.2.2]octa-7-ene with thiourea to produce (2S, 3S, 4R)-4-hydroxy-2,3-O-isopropylidenecyclohex-5-enone;
(c) treating said (2S, 3S, 4R)- 4-hydroxy-2,3-O-isopropylidenecyclohex-5-enone with t-butyldimethylchlorosilane to produce (2S, 3S, 4R)-4-t-butyldimethylsilyloxy-2,3-O-isopropylidene-5-cyclohexanone;
(d) hydrogenating said (2S, 3S, 4R)-4-t-butyldimethylsilyloxy-2,3-O-isopropylidene-5-cyclohexanone to produce (2S, 3S, 4R)-2,3-O-isopropylidene-4-t-butyldimethylsilyloxy-cyclohexanone;
(e) treating said (2S, 3S, 4R)-2,3-O-isopropylidene-4-t-butyldimethylsilyloxy-cyclohexanone with L-selectride to produce (1R, 2R, 3R, 4R)-1-hydroxy-2,3-di-O-(t-butyl)dimethylsiloloxy-cyclohexane; and
(f) treating said (1R, 2R, 3R, 4R)-1-hydroxy-2,3-di-O-(t-butyl)dimethylsilyloxy-cyclohexane with a mixture of water and acetone to produce Conduritol C.

14. A method of producing Conduritol F, said method comprising the steps of:
(a) treating (2R, 3S)-2,3-O-isopropylidene-1-chlorocyclohexa-4,6-diene with m-chloroperbenzoic acid to produce (1R, 2S, 3S, 6R)-4-chloro-2,3-di-O-isopropylidene-7-oxa-bicyclo[4.1.0]heptane;
(b) treating said (1R, 2S, 3S, 6R)-4-chloro-2,3-di-O-isopropylidene-7-oxa-bicyclo[4.1.0]heptane with acidified water to produce (2S, 3S, 4R, 5S)-1-chloro-2,3-O-isopropylidene-4,5-dihydroxy-cyclohex-6-ene;
(c) reducing said (2S, 3S, 4R, 5S)-1-chloro-2,3-O-isopropylidene-4,5-dihydroxy-cyclohex-6-ene to produce (2S, 3S, 4R, 5S)-2,3-O-isopropylidene-4,5-dihydroxy-cyclohex-6-ene; and
(d) deprotecting said (2S, 3S, 4R, 5S)-2,3-O-isopropylidene-4,5-dihydroxy-cyclohex-6-ene to produce Conduritol F.

15. A method of producing (+)-Pinitol, said method comprising the steps of:
(a) treating (2R, 3S)-2,3-O-isopropylidene-1-chlorocyclohexa-4,6-diene with m-chloroperbenzoic acid to produce (1R, 2S, 3S, 6R)-4-chloro-2,3-di-O-isopropylidene-7-oxa-bicyclo[4.1.0]heptane;
(b) treating said (1R, 2S, 3S, 6R)-4-chloro-2,3-di-O-isopropylidene-7-oxa-bicyclo[4.1.0]heptane with acidified methanol to produce (1S, 2R, 3S, 4S)-5-chlorocyclohex-5-ene-1-O-methyl-2-hydroxy-3,4-di-O-acetonide;
(c) reducing said (1S, 2R, 3S, 4S)-5-chlorocyclohex-5-ene-1-O-methyl-2-hydroxy-3,4-di-O-acetonide to produce (2S, 3S, 4R, 5S)-2,3-O-isopropylidene-4-hydroxy-5-methoxy-cyclohex-6-ene; and
(d) treating said (2S, 3S, 4R, 5S)-2,3-O-isopropylidene-4-hydroxy-5-methoxy-cyclohex-6-ene with osmium tetroxide to produce (+)-Pinitol.

16. A method of producing (-)-Pinitol, said method comprising the steps of:
(a) treating (2R, 3S)-2,3-O-isopropylidene-1-chlorocyclohexa-4,6-diene with osmium tetroxide to produce (2S, 3S, 4R, 5R)-1-chloro-2,3-O-isopropylidene-4,5-dihydroxy-cyclohex-6-ene;
(b) reducing said (2S, 3S, 4R, 5R)-1-chloro-2,3-O-isopropylidene-4,5-dihydroxy-cyclohex-6-ene;
(c) treating said (2S, 3S, 4R, 5R)-2,3-O-isopropylidene-4,5-dihydroxy-cyclohex-6-ene with m-chloroperbenzoic acid to produce an epoxide; and
(d) treating said epoxide with methanol and acid to produce (-)-Pinitol.

17. An intermediate compound useful in the synthesis of cyclitols, said intermediate being selected from the group consisting of:
(1S,2R,3R,4R)-1,2-Dihydroxy-3,4-di-O-isopropylidene cyclohex-5-ene; (1R,2R,3S,4R)-1,2-dihydroxy-3,4-di-O-isopropylidene cyclohex-5-ene; (1S,2R,3R,4S,5R,6S)-2,3-Dihydroxy-4,5-di-O-isopropylidene-7-oxa-bicyclo[4.1.0]heptane; (1R,2R,3R,4S,5R,6S)-2,3,6-trihydroxy-4,5-di-O-isopropylidene-1-O-methylcyclohexane; (3R,4R,5S,6S)-1-Chloro-4-hydroxy-5,6-di-O-isopropylidene-3-O-methylcyclohex-1-ene; (3R,4R,5S,6S)-1-Bromo-4-hydroxy-5,6-di-O-isopropylidene-3-O-methylcyclohex-1-ene; (3R,4R,5S,6S)-3-O-Methyl-4-hydroxy-5,6-di-O-isopropylidene cyclohex-1-ene; (4R,5S,6R)-4-O-(t-butyldimethylsilyl)-5,6-di-O-isopropylidenelcyclohex-2-en-1-one; (2S,3S,4R)-4-O-(t-butyldimethylsily)-2,3-di-O-isopropylidenecyclohexan-1-one; (1R,2R,3R,4R)-1-hydroxy-2,3-di-O-isopropylidene-4-O-(t-butyldimethylsily)cyclohexane; and (1R,2R,3R,4R)-1-Hydroxy-2,3-di-O-isopropylidene-4-O-(t-butyldimethylsilyl)cyclohex-5-ene.

## Patentansprüche

1. Verfahren zur Herstellung eines Diols der Formel 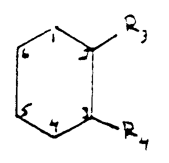 worin R₃ und R₄ Hydroxyl oder Alkoxy sind, wobei das Verfahren die folgenden Schritte umfaßt:
(a) Bereitstellen eines substituierten Arendiols der Formel 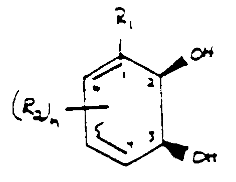 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols; und
(c) vollständiges Reduzieren des geschützten substituierten Arendiols mit geeigneten Reagentien, um R₃ und R₄ zu liefern.

2. Verfahren zur Herstellung eines Triols der Formel 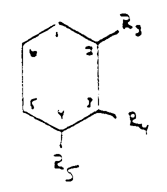 worin R₃, R₄ und R₅ Hydroxyl oder Alkoxy sind, wobei das Verfahren die folgenden Schritte umfaßt:
(a) Bereitstellen eines substituierten Arendiols der Formel 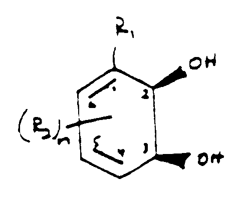 worin R₁ Halogen, Niederalkyl oder Niederalkenyl ist, R₂ Halogen oder eine Brückengruppe ist und n gleich 0 - 3 ist;
(b) Schützen der Diol-Funktionalität des substituierten Arendiols;
(c) Bilden eines Epoxids zwischen den Kohlenstoffatomen 4 und 5 des geschützten substituierten Arendiols;
(d) Behandeln des Epoxids mit angesäuertem Wasser oder einem geeigneten angesäuerten Alkohol, um ein Enon mit R₅ zu erhalten; und
(e) Reduzieren der Funktionalität an Kohlenstoff 1 des Enons zu Wasserstoff.

3. Verfahren zur Herstellung eines Triols der Formel 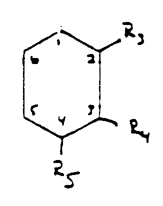 worin R₃, R₄ und R₅ Hydroxyl oder Alkoxy sind, wobei das Verfahren die folgenden Schritte umfaßt:
(a) Bereitstellen eines substituierten Arendiols der Formel 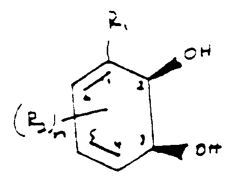 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols;
(c) Bilden eines Endoperoxids zwischen den Kohlenstoffatomen 1 und 4 des geschützten substituierten Arendiols durch Behandlung mit Singulett-Sauerstoff;
(d) Behandeln des Endoperoxids mit Thioharnstoff, um ein Enon zu erhalten; und
(e) Reduzieren der Funktionalität an Kohlenstoff 1 des Enons zu Wasserstoff.

4. Verfahren zur Herstellung eines Tetrols der Formel 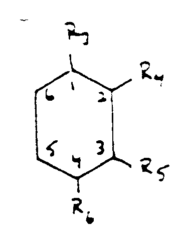 worin R₃, R₄, R₅ und R₆ Hydroxyl oder Alkoxy sind, wobei das Verfahren die folgenden Schritte umfaßt:
(a) Bereitstellen eines substituierten Arendiols der Formel 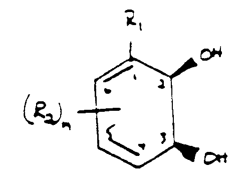 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols;
(c) Bilden eines Endoperoxids zwischen den Kohlenstoffatomen 1 und 4 des geschützten substituierten Arendiols durch Behandlung mit Singulett-Sauerstoff;
(d) Behandeln des Endoperoxids mit Thioharnstoff, um ein Enon zu erhalten; und
(e) Reduzieren der Funktionalität an Kohlenstoff 1 des Enons, um R₃ herzustellen.

5. Verfahren zur Herstellung eines Tetrols der Formel 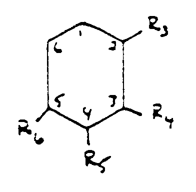 worin R₃, R₄, R₅ und R₆ Hydroxyl oder Alkoxy sind, wobei das Verfahren die folgenden Schritte umfaßt:
(a) Bereitstellen eines substituierten Arendiols der Formel 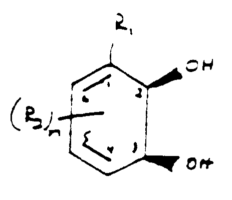 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols;
(c) Umwandeln des geschützten substituierten Arendiols zu einem Enon mit der Keto-Funktionalität an Kohlenstoff 1 und R₅ an Kohlenstoff 4;
(d) Bilden eines Epoxids zwischen den Kohlenstoffatomen 4 und 5 des Enons;
(e) Behandeln des Epoxids mit angesäuertem Wasser oder einem geeigneten angesäuerten Alkohol, um R₆ zu erhalten; und
(f) Reduzieren der Funktionalität an Kohlenstoff 1 zu Wasserstoff.

6. Verfahren zur Herstellung eines Tetrols der Formel 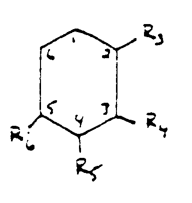 worin R₃, R₄, R₅ und R₆ Hydroxyl oder Alkoxy sind, wobei das Verfahren die folgenden Schritte umfaßt:
(a) Bereitstellen eines substituierten Arendiols der Formel 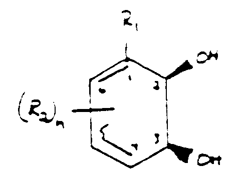 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols;
(c) Bilden eines Epoxids zwischen den Kohlenstoffatomen 4 und 5 des geschützten substituierten Arendiols;
(d) Behandeln des Epoxids mit angesäuertem Wasser oder einem geeigneten angesäuerten Alkohol, um R₅ und R₆ zu erhalten; und
(e) Reduzieren der Funktionalität an Kohlenstoff 1 zu Wasserstoff.

7. Verfahren zur Herstellung eines Pentols der Formel 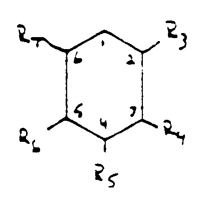 worin R₃, R₄, R₅, R₆ und R₇ Hydroxyl oder Alkoxy sind, wobei das Verfahren die folgenden Schritte umfaßt:
(a) Bereitstellen eines substituierten Arendiols der Formel 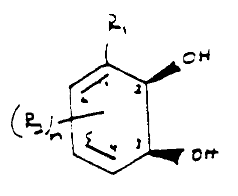 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols;
(c) Bilden eines Epoxids zwischen den Kohlenstoffatomen 5 und 6 des geschützten substituierten Arendiols;
(d) Behandeln des Epoxids mit angesäuertem Wasser oder einem geeigneten angesäuerten Alkohol, um R₆ und R₇ zu erhalten; und
(e) Reduzieren der Funktionalität an Kohlenstoff 1 zu Wasserstoff.

8. Verfahren zur Herstellung eines Pentols der Formel 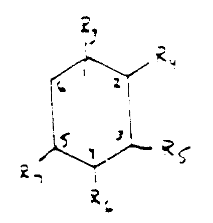 worin R₃, R₄, R₅, R₆ und R₇ Hydroxyl oder Alkoxy sind, wobei das Verfahren die folgenden Schritte umfaßt:
(a) Bereitstellen eines substituierten Arendiols der Formel 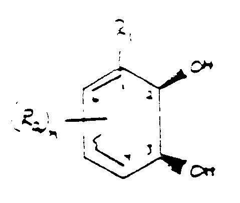 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols;
(c) Bilden eines Epoxids zwischen den Kohlenstoffatomen 5 und 6 des geschützten substituierten Arendiols;
(d) Behandeln des Epoxids mit angesäuertem Wasser oder einem geeigneten angesäuerten Alkohol, um R₇ zu erhalten; und
(e) Reduzieren der Funktionalität an Kohlenstoff 1, um R₃ herzustellen.

9. Verfahren zur Herstellung eines Hexols der Formel 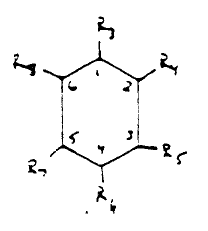 worin R₃, R₄, R₅, R₆, R₇ und R₈ Hydroxyl oder Alkoxy sind, wobei das Verfahren die folgenden Schritte umfaßt:
(a) Bereitstellen eines substituierten Arendiols der Formel 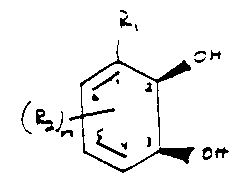 worin R₁ Halogen, Niederalkyl oder Niederalkenyl ist, R₂ Halogen oder eine Brückengruppe ist und n 0 - 3 ist;
(b) Schützen der Diol-Funktionalität des substituierten Arendiols;
(c) Bilden eines Epoxids zwischen den Kohlenstoffatomen 4 und 5 des geschützten substituierten Arendiols;
(d) Behandeln des Epoxids mit angesäuertem Wasser oder einem geeigneten angesäuerten Alkohol, um ein geschütztes Tetrol mit R₆ und R₇ zu erhalten;
(e) Reduzieren der Funktionalität an Kohlenstoff 1 zu Wasserstoff; und
(f) entweder (1) Bilden eines Epoxids zwischen den Kohlenstoffatomen 1 und 6 des geschützten Tetrols und Behandeln des Epoxids mit angesäuertem Wasser oder einem geeigneten angesäuerten Alkohol, um R₃ und R₈ zu erhalten, oder (2) Behandeln des reduzierten geschützten Tetrols mit Osmiumtetroxid, um R₃ und R₈ zu erhalten.

10. Verfahren nach Anspruch 1, 2, 3, 4, 5, 6, 7, 8 oder 9, worin das substituierte Arendiol der Formel 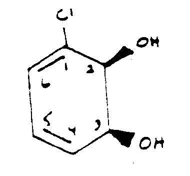 entspricht.

11. Verfahren nach Anspruch 1, 2, 3, 4, 5, 6, 7, 8 oder 9, worin eines oder mehrere als Zwischenprodukt im Verfahren verwendete Cyclitole vor dem Fortfahren mit den nachfolgenden Schritten des Verfahrens in ein Phosphat-Derivat umgewandelt werden.

12. Verfahren nach Anspruch 1, 2, 3, 4, 5, 6, 7, 8 oder 9, worin ein oder mehrere im Verfahren verwendete Reagentien radioaktiv sind und worin das nach dem Verfahren hergestellte Cyclitol radioaktiv ist.

13. Verfahren zur Herstellung von Conduritol C, wobei das Verfahren die folgenden Schritte umfaßt:
(a) Behandeln von (2R, 3S)-2,3-O-Isopropyliden-1-chlorcyclohexa-4,6-dien mit Singulett-Sauerstoff, um (1S, 2S, 3S, 4R)-1-Chlor-2,3-O-isopropyliden-5,6-dioxabicyclo[2.2.2]octa-7-en herzustellen;
(b) Behandeln des (1S, 2S, 3S, 4R)-1-Chlor-2,3-O-isopropyliden-5,6-dioxabicyclo[2.2.2]octa-7-ens mit Thioharnstoff, um (2S, 3S, 4R)-4-Hydroxy-2,3-O-isopropylidencyclohex-5-enon herzustellen;
(c) Behandeln des (2S, 3S, 4R)-4-Hydroxy-2,3-O-isopropylidencyclohex-5-enons mit t-Butyldimethylchlorsilan, um (2S, 3S, 4R)-4-t-Butyldimethylsilyloxy-2,3-O-isopropyliden-5-cyclohexanon herzustellen;
(d) Hydrieren des (2S, 3S, 4R)-4-t-Butyldimethylsilyloxy-2,3-O-isopropyliden-5-cyclohexanons, um (2S, 3S, 4R)-2,3-O-Isopropyliden-4-t-butyldimethylsilyloxy-cyclohexanon herzustellen;
(e) Behandeln des (2S, 3S, 4R)-2,3-O-Isopropyliden-4-t-butyldimethylsilyloxy-cyclohexanons mit L-Selectrid, um (1R, 2R, 3R, 4R)-1-Hydroxy-2,3-di-O-(t-butyl)dimethylsilyloxy-cyclohexan herzustellen; und
(f) Behandeln des (1R, 2R, 3R, 4R)-1-Hydroxy-2,3-di-O-(t-butyl)dimethylsilyloxy-cyclohexans mit einem Gemisch aus Wasser und Aceton, um Conduritol C herzustellen.

14. Verfahren zur Herstellung von Conduritol F., wobei das Verfahren die folgenden Schritte umfaßt:
(a) Behandeln von (2R, 3S)-2,3-O-Isopropyliden-1-chlorcyclohexa-4,6-dien mit m-Chlorperbenzoesäure, um (1R, 2S, 3S, 6R)-4-Chlor-2,3-di-O-isopropyliden-7-oxa-bicyclo[4.1.0]heptan herzustellen;
(b) Behandeln des (1R, 2S, 3S, 6R)-4-Chlor-2,3-di-O-isopropyliden-7-oxa-bicyclo[4.1.0]heptans mit angesäuertem Wasser, um (2S, 3S, 4R, 5S)-1-Chlor-2,3-O-isopropyliden-4,5-dihydroxy-cyclohex-6-en herzustellen;
(c) Reduzieren des (2S, 3S, 4R, 5S)-1-Chlor-2,3-O-isopropyliden-4,5-dihydroxy-cyclohex-6-ens, um (2S, 3S, 4R, 5S)-2,3-O-Isopropyliden-4,5-dihydroxy-cyclohex-6-en herzustellen; und
(d) Abspalten der Schutzgruppe von (2S, 3S, 4R, 5S)-2,3-O-Isopropyliden-4,5-dihydroxy-cyclohex-6-en, um Conduritol F herzustellen.

15. Verfahren zur Herstellung von (+)-Pinitol, wobei das Verfahren die folgenden Schritte umfaßt:
(a) Behandeln von (2R, 3S)-2,3-O-Isopropyliden-1-chlorcyclohexa-4,6-dien mit m-Chlorperbenzoesäure, um (1R, 2S, 3S, 6R)-4-Chlor-2,3-di-O-isopropyliden-7-oxa-bicyclo[4.1.0]heptan herzustellen;
(b) Behandeln des (1R, 2S, 3S, 6R)-4-Chlor-2,3-di-O-isopropyliden-7-oxa-bicyclo[4.1.0]heptans mit angesäuertem Methanol, um (1S, 2R, 3S, 4S)-5-Chlorcyclohex-5-en-1-O-methyl-2-hydroxy-3,4-di-O-acetonid herzustellen;
(c) Reduzieren des (1S, 2R, 3S, 4S)-5-Chlorcyclohex-5-en-1-O-methyl-2-hydroxy-3,4-di-O-acetonids, um (2S, 3S, 4R, 5S)-2,3-O-Isopropyliden-4-hydroxy-5-methoxy-cyclohex-6-en herzustellen; und
(d) Behandeln des (2S, 3S, 4R, 5S)-2,3-O-Isopropyliden-4-hydroxy-5-methoxy-cyclohex-6-ens mit Osmiumtetroxid, um (+)-Pinitol herzustellen.

16. Verfahren zur Herstellung von (-)-Pinitol, wobei das Verfahren die folgenden Schritte umfaßt:
(a) Behandeln von (2R, 3S)-2,3-O-Isopropyliden-1-chlorcyclohexa-4,6-dien mit Osmiumtetroxid, um (2S, 3S, 4R, 5R)-1-Chlor-2,3-O-isopropyliden-4,5-dihydroxy-cyclohex-6-en herzustellen;
(b) Reduzieren des (2S, 3S, 4R, 5R)-1-Chlor-2,3-O-isopropyliden-4,5-dihydroxy-cyclohex-6-ens;
(c) Behandeln des (2S, 3S, 4R, 5R)-2,3-O-Isopropyliden-4,5-dihydroxy-cyclohex-6-ens mit m-Chlorperbenzoesäure, um ein Epoxid herzustellen; und
(d) Behandeln des Epoxids mit Methanol und Säure, um (-)-Pinitol herzustellen.

17. Verbindung, die als Zwischenprodukt bei der Synthese von Cyclitolen nützlich sind, wobei das Zwischenprodukt ausgewählt wird aus:
(1S, 2R, 3R, 4R)-1,2-Dihydroxy-3,4-di-O-isopropyliden-cyclohex-5-en; (1R, 2R, 3S, 4R)-1,2-Dihydroxy-3,4-di-O-isopropyliden-cyclohex-5-en; (1S, 2R, 3R, 4S, 5R, 6S)-2,3-Dihydroxy-4,5-di-O-isopropyliden-7-oxa-bicyclo[4.1.0]heptan; (1R, 2R, 3R, 4S, 5R, 6S)-2,3,6-Trihydroxy-4,5-di-O-isopropyliden-1-O-methylcyclohexan; (3R, 4R, 5S, 6S)-1-Chlor-4-hydroxy-5,6-di-O-isopropyliden-3-O-methylcyclohex-1-en; (3R, 4R, 5S, 6S)-1-Brom-4-hydroxy-5,6-di-O-isopropyliden-3-O-methylcyclohex-1-en; (3R, 4R, 5S, 6S)-3-O-Methyl-4-hydroxy-5,6-di-O-isopropyliden-cyclohex-1-en; (4R, 5S, 6R)-4-O-(t-Butyl-dimethylsilyl)-5,6-di-O-isopropyliden-cyclohex-2-en-1-on; (2S, 3S, 4R)-4-O-(t-Butyl-dimethylsilyl)-2,3-di-O-isopropyliden-cyclohexan-1-on; (1R, 2R, 3R, 4R)-1-Hydroxy-2,3-di-O-isopropyliden-4-O-(t-butyldimethylsilyl)cyclohexan; und (1R, 2R, 3R, 4R)-1-Hydroxy-2,3-di-O-isopropyliden-4-O-(t-butyldimethylsilyl)cyclohex-5-en.

## Revendications

1. Procédé de préparation d'un diol répondant à la formule 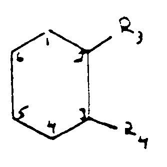 où R³ et R⁴ sont un hydroxyle ou un alcoxy, ledit procédé comprenant les étapes consistant à :
(a) fournir un arènediol substitué répondant à la formule 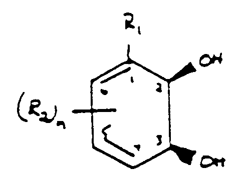 où R¹ est un halogène, un alkyle inférieur ou un alcényle inférieur, R² est un halogène ou un groupe de pontage ; et n vaut de 0 à 3 ;
(b) protéger la fonction diol dudit arènediol substitué ;
(c) réduire complètement ledit arènediol substitué protégé avec des réactifs appropriés pour fournir R³ et R⁴.

2. Procédé de production d'un triol répondant à la formule 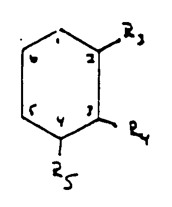 où R³, R⁴ et R⁵ sont un hydroxyle ou un alcoxy, ledit procédé comprenant les étapes consistant à :
(a) fournir un arènediol substitué répondant à la formule 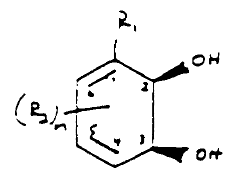 où R¹ est un halogène, un alkyle inférieur ou un alcényle inférieur, R² est un halogène ou un groupe de pontage et n vaut de 0 à 3 ;
(b) protéger la fonction diol dudit arènediol substitué ;
(c) former un époxyde entre les carbones 4 et 5 dudit arènediol substitué protégé ;
(d) traiter ledit époxyde avec de l'eau acidifiée ou un alcool acidifié approprié pour fournir une énone ayant R⁵ ; et
(e) réduire la fonctionalité au carbone 1 de ladite énone en l'hydrogène.

3. Procédé de préparation d'un triol répondant à la formule 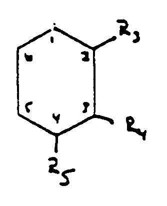 où R³, R⁴ et R⁵ sont un hydroxyle ou un alcoxy, ledit procédé comprenant les étapes consistant à :
(a) fournir un arène diol substitué répondant à la formule 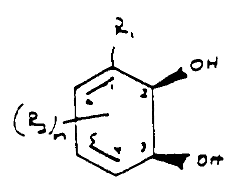 où R¹ est un halogène, un alkyle inférieur ou un alcényle inférieur, R² est un halogène ou un groupe de pontage et n vaut de 0 à 3 ;
(b) protéger la fonction diol dudit arènediol substitué ;
(c) former un endopérioxyde entre les carbones 1 et 4 dudit arènediol substitué protégé, par traitement avec de l'oxygène singulet ;
(d) traiter ledit endopérioxyde avec de la thiourée pour former une énone ; et
(e) réduire la fonctionalité au carbone 1 de ladite énone en l'hydrogène.

4. Procédé de préparation d'un tétrol répondant à la formule 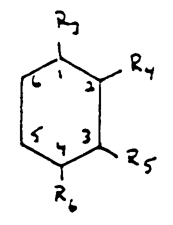 où R³, R⁴, R⁵ et R⁶ sont un hydroxyle ou un alcoxy, ledit procédé comprenant les étapes consistant à :
(a) fournir un arènediol substitué répondant à la formule 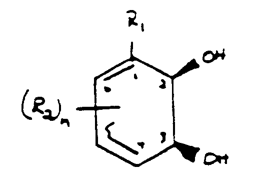 où R¹ est un halogène, un alkyle inférieur ou un alcényle inférieur, R² est un halogène ou un groupe de pontage et n vaut de 0 à 3 ;
(b) protéger la fonction diol dudit arènediol substitué ;
(c) former un endopérioxyde entre les carbones 1 et 4 dudit arènediol substitué protégé, par traitement avec de l'oxygène singulet ;
(d) traiter ledit endopérioxyde avec de la thiourée pour former une énone ; et
(e) réduire la fonction au carbone 1 de ladite énone pour produire R³.

5. Procédé de préparation d'un tétrol répondant à la formule 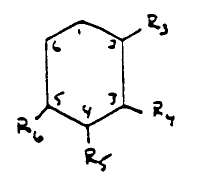 où R³, R⁴, R⁵ et R⁶ sont un hydroxyle ou un alcoxy, ledit procédé comprenant les étapes consistant à :
(a) fournir un arènediol substitué répondant à la formule 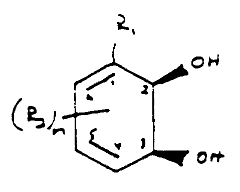 où R¹ est un halogène, un alkyle inférieur ou un alcényle inférieur, R² et un halogène ou un groupe de pontage et n vaut de 0 à 3 ;
(b) protéger la fonction diol dudit arènediol substitué ;
(c) convertir ledit arènediol substitué protégé en une énone ayant la fonction céto au carbone 1 et R⁵ au carbone 4 ;
(d) former un époxyde entre les carbones 4 et 5 de ladite énone ;
(e) traiter ledit époxyde avec de l'eau acidifiée ou un alcool acidifié approprié pour fournir R⁶ ; et
(f) réduire la fonction au carbone 1 en l'hydrogène.

6. Procédé de préparation d'un tétrol répondant à la formule 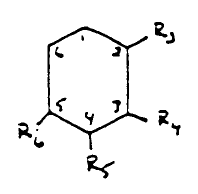 où R³, R⁴, R⁵ et R⁶ sont un hydroxyle ou un alcoxy, ledit procédé comprenant les étapes consistant à :
(a) fournir un arènediol substitué répondant à la formule 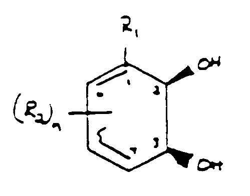 où R¹ est un halogène, un alkyle inférieur ou un alcényle inférieur, R² est un halogène ou un groupe de pontage et n vaut de 0 à 3 ;
(b) protéger la fonction diol dudit arènediol substitué ;
(c) former un époxyde entre les carbones 4 et 5 dudit arènediol substitué protégé ;
(d) traiter ledit époxyde avec de l'eau acidifiée ou un alcool acidifié approprié pour fournir R⁵ et R⁶ ; et
(e) réduire la fonctionalité au carbone 1 en l'hydrogène.

7. Procédé de préparation d'un pentol répondant à la formule 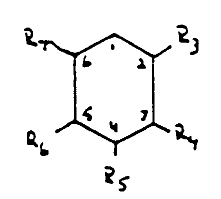 où R³, R⁴, R⁵, R⁶ et R⁷ sont un hydroxyle ou un alcoxy, ledit procédé conprenant les étapes consistant à :
(a) fournir un arènediol substitué répondant à la formule 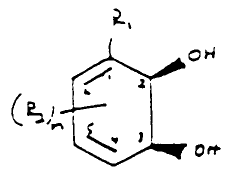 où R¹ est un halogène, un alkyle inférieur ou un alcényle inférieur, R² est un halogène ou un groupe de pontage et n vaut de 0 à 3 ;
(b) protéger la fonction diol dudit arènediol substitué ;
(c) former un époxyde entre les carbones 5 et 6 dudit arènediol substitué protégé ;
(d) traiter ledit époxyde avec de l'eau acidifiée ou un alcool acidifié approprié pour fournir R⁶ et R⁷ ; et
(e) réduire la fonction au carbone 1 en l'hydrogène.

8. Procédé de préparation d'un pentol répondant à la formule 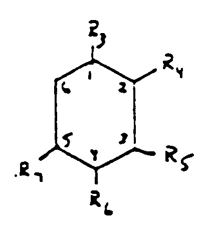 où R³, R⁴, R⁵, R⁶ et R⁷ sont un hydroxyle ou un alcoxy, ledit procédé comprenant les étapes consistant à :
(a) fournir un arènediol substitué répondant à la formule 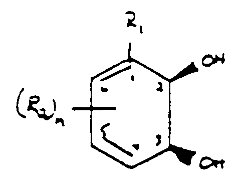 où R¹ est un halogène, un alkyle inférieur ou un alcényle inférieur, R² est un halogène ou un groupe de pontage et n vaut de 0 à 3.
(b) protéger la fonction diol dudit arènediol substitué ;
(c) former un époxyde entre les carbones 5 et 6 dudit arènediol substitué protégé ;
(d) traiter ledit époxyde avec de l'eau acidifiée ou un alcool acidifié approprié pour fournir R⁷ ; et
(e) réduire la fonction au carbone 1 pour produire R³.

9. Procédé de préparation d'un hexol répondant à la formule 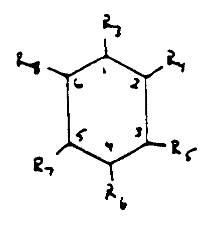 où R³, R⁴, R⁵, R⁶, R⁷ et R⁸ sont un hydroxyle ou un alcoxy, ledit procédé comprenant les étapes consistant à :
(a) fournir un arènediol substitué répondant à la formule 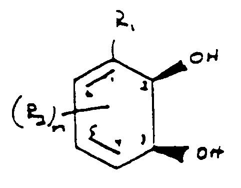 où R¹ est un halogène, un alkyle inférieur ou un alcényle inférieur, R² est un halogène ou un groupe de pontage et n vaut de 0 à 3 ;
(b) protéger la fonction diol dudit arènediol substitué ;
(c) former un époxyde entre les carbones 4 et 5 dudit arènediol substitué protégé ;
(d) traiter ledit époxyde avec de l'eau acidifiée ou un alcool acidifié approprié pour fournir un tétrol protégé ayant R⁶ et R⁷ ;
(e) réduire la fonction au carbone 1 dudit tétrol protégé en l'hydrogène ; et
(f) soit (1) former un époxyde entre les carbones 1 et 6 dudit tétrol protégé et traiter ledit époxyde avec de l'eau acidifiée ou un alcool acidifié approprié pour fournir R³ et R⁸, soit (2) traiter ledit tétrol protégé réduit avec du tétraoxyde d'osmium pour fournir R³ et R⁸.

10. Procédé de la revendication 1, 2, 3, 4, 5, 6, 7, 8 ou 9 dans lequel ledit arènediol substitué répond à la formule

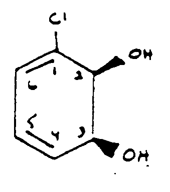

11. Procédé de la revendication 1, 2, 3, 4, 5, 6, 7, 8 ou 9 dans lequel au moins un cyclitol intermédiaire employé dans le procédé est converti en un dérivé phosphate avant de procéder aux étapes subséquentes du procédé.

12. Procédé de la revendication 1, 2, 3, 4, 5, 6, 7, 8 ou 9 dans lequel au moins un réactif de ceux utilisés dans le procédé est radioactif et dans lequel le cyclitol produit selon ledit procédé est radioactif.

13. Procédé de préparation de Conduritol C, ledit procédé comprenant les étapes consistant à :
(a) traiter le (2R,3S)-2,3-O-isopropylidène-1-chlorocyclohexa-4,6-diène avec de l'oxygène singulet pour produire du (1S,2S,3S,4R)-1-chloro-2,3-O-isopropylidène-5,6-dioxabicyclo[2.2.2]octa-7-ène ;
(b) traiter ledit (1S,2S,3S,4R)-1-chloro-2,3-O-isopropylidène-5,6-dioxabicyclo[2.2.2]octa-7-ène avec de la thio-urée pour produire de la (2S,3S,4R)-4-hydroxy-2,3-O-isopropylidènecyclohex-5-énone ;
(c) traiter ladite (2S,3S,4R)-4-hydroxy-2,3-O-isopropylidènecyclohex-5-énone avec du t-butyldiméthylchloro-silane pour produire de la (2S,3S,4R)-4-t-butyldiméthylsilyl-oxy-2,3-O-isopropoylidène-5-cyclohexanone ;
(d) hydrogéner ladite (2S,3S,4R)-4-t-butyl-diméthylsilyloxy-2,3-O-isopropylidène-5-cyclohexanone pour produire de la (2S,3S,4R)-2,3-O-isopropylidène-4-t-butyl-diméthylsilyloxy-cyclohexanone ;
(e) traiter ladite (2S,3S,4R)-2,3-O-isopropylidène-4-t-butyldiméthylsilyloxy-cyclohexanone avec du L-sélectride pour produire du (1R,2R,3R,4R)-1-hydroxy-2,3-di-O-(t-butyl)diméthylsiloloxy-cyclohexane ; et
(f) traiter ledit (1R,2R,3R,4R)-1-hydroxy-2,3-di-O-(t-butyl)diméthylsilyloxy-cyclohexane avec de l'eau et de l'acétone en mélange pour produire du Conduritol C.

14. Procédé de préparation de Conduritol F, ledit procédé comprenant les étapes consistant à :
(a) traiter le (2R,3S)-2,3-O-isopropylidène-1-chlorocyclohexa-4,6-diène avec de l'acide m-chloroperbenzoïque pour produire du (1R,2S,3S,6R)-4-chloro-2,3-di-O-isopropylidène-7-oxabicyclo[4.1.0]heptane ;
(b) traiter ledit (1R,2S,3S,6R)-4-chloro-2,3-di-O-isopropylidène-7-oxabicyclo[4.1.0]heptane avec de l'eau acidifiée pour produire du (2S,3S,4R,5S)-1-chloro-2,3-O-isopropylidène-4,5-dihydroxy-cyclohex-6-ène ;
(c) réduire ledit (2S,3S,4R,5S)-1-chloro-2,3-O-isopropylidène-4,5-dihydroxy-cyclohex-6-ène pour produire du (2S,3S,4R,5S)-2,3-O-isopropylidène-4,5-dihydroxy-cyclohex-6-ène ; et
(d) déprotéger ledit (2S,3S,4R,5S)-2,3-O-isopropylidène-4,5-dihydroxy-cyclohex-6-ène pour produire du Conduritol F.

15. Procédé de préparation de Pinitol (+), ledit procédé comprenant les étapes consistant à :
(a) traiter le (2R,3S)-2,3-O-isopropylidène-1-chlorocyclohexa-4,6-diène avec de l'acide m-chloroperbenzoïque pour produire du (1R,2S,3S,6R)-4-chloro-2,3-di-O-isopropylidène-7-oxabicyclo[4.1.0]heptane ;
(b) traiter ledit (1R,2S,3S,6R)-4-chloro-2,3-di-O-isopropylidène-7-oxa-bicyclo[4.1.0]heptane avec du méthanol acidifié pour produire du (1S,2R,3S,4S)-5-chlorocyclohex-5-ène-1-O-méthyl-2-hydroxy-3,4-di-O-acétonide.
(c) réduire ledit (1S,2R,3S,4S)-5-chlorocyclohex-5-ène-1-O-méthyl-2-hydroxy-3,4-di-O-acétonide pour produire du (2S,3S,4R,5S)-2,3-O-isopropylidène-4-hydroxy-5-méthoxy-cyclohex-6-ène ; et
(d) traiter ledit (2S,3S,4R,5S)-2,3-O-isopropylidène-4-hydroxy-5-méthoxy-cyclohex-6-ène avec du tétraoxyde d'osmium pour produire du Pinitol (+).

16. Procédé de préparation de Pinitol (-), ledit procédé comprenant les étapes consistant à :
(a) traiter le (2R,3S)-2,3-O-isopropylidène-1-chlorocyclohexa-4,6-diène avec du tétraoxyde d'osmium pour produire du (2S,3S,4R,5R)-1-chloro-2,3-O-isopropylidène-4,5-dihydroxy-cyclohex-6-ène ;
(b) réduire ledit (2S,3S,4R,5R)-1-chloro-2,3-O-isopropylidène-4,5-dihydroxy-cyclohex-6-ène ;
(c) traiter ledit (2S,3S,4R,5R)-2,3-O-isopropylidène-4,5-dihydroxy-cyclohex-6-ène avec de l'acide m-chloroperbenzoïque pour produire de l'époxyde ; et
(d) traiter ledit époxyde avec du méthanol et de l'acide pour produire du Pinitol (-).

17. Composé intermédiaire utile dans la synthèse des cyclitols, ledit intermédiaire étant choisi parmi le groupe constitué par :
(1S,2R,3R,4R)-1,2-dihydroxy-3,4-di-O-isopropylidène cyclohex-5-ène ; (1R,2R,3S,4R)-1,2-dihydroxy-3,4-di-O-isopropylidène cyclohex-5-ène ; (1S,2R,3R,4S,5R,6S)-2,3-dihydroxy-4,5-di-O-isopropylidène-7-oxa-bicyclo[4.1.0]heptane ; (1R,2R,3R,4S,5R,6S)-2,3,6-trihydroxy-4,5-di-O-isopropylidène-1-O-méthylcyclohexane ; (3R,4R,5S,6S)-1-chloro-4-hydroxy-5,6-di-O-isopropylidène-3-O-méthylcyclohex-1-ène ; (3R,4R,5S,6S)-1-bromo-4-hydroxy-5,6-di-O-isopropylidène-3-O-méthylcyclohex-1-ène ; (3R,4R,5S,6S)-3-O-méthyl-4-hydroxy-5,6-di-O-isopropylidène cyclohex-1-ène ; (4R,5S,6R)-4-O-(t-butyldiméthylsilyl)-5,6-di-O-isopropylidènecyclohex-2-én-1-one ; (2S,3S,4R)-4-O-(t-butyldiméthylsilyl)-2,3-di-O-isopropylidènecyclohexan-1-one ; (1R,2R,3R,4R)-1-hydroxy-2,3-di-O-isopropylidène-4-O-(t-butyldiméthylsilyl)cyclohexane ; et (1R,2R,3R,4R)-1-hydroxy-2,3-di-O-isopropylidène-4-O-(t-butyldiméthylsilyl)cyclohex-5-ène.
